# EUROPEAN PATENT SPECIFICATION

(11) **EP 0 474 800 B1**
(45) Date of publication and mention of the grant of the patent: **12.01.1994**
(21) Application number: 91903310.0
(22) Date of filing: 08.02.1991
(51) Int. Cl.: A61K 31/71, A61K 9/08, A61K 47/32

(54) **EYE-DROP**
AUGENTROPFEN
GOUTTES POUR LES YEUX

(30) Priority: 15.02.1990 HU 80890
(43) Date of publication of application: 18.03.1992
(73) Proprietor: CHINOIN Gyògyszer és Vegyészeti Termékek Gyára RT., H-1045 Budapest IV (HU)
(72) Inventor: Z.SZABO, Anna, H-1163 Budapest (HU)
(74) Representative: Beetz & Partner Patentanwälte
(86) International application number: HU9100006
(87) International publication number: WO9112008

(56) References cited:
- EP-A- 0 224 868
- WO-A-85/05621
- AT-B- 344 885
- CH-A- 543 278
- DE-A- 2 615 140
- DE-A- 3 104 282
- GB-A- 1 340 518
- GB-A- 1 512 604

## Description

The invention relates to a stable aqueous solution containing as active ingredient primycin.

Subjects of the present invention are further therapeutical compositions prepared from the stable aqueous solution.

Primycin is a macrolide antibiotic agent, which is characterized in the literature (J. Chem. Soc. Perkin I. 1974, p. 816) by a single formula:
/5-(18-(α-D-arabinofuranosyloxy)-2-butyl-3,7,11,15,19,21,23,25,27-nonahydroxy-4,16,32,34-tetramethyl-1-oxo-oxacyclohexatriakonta-16,32-diene-35-yl-)/4-hydroxyhexyl/guanidium-sulphate.
Although the above literature describes its structure by a single formula, it is known, that it is an antibiotic-mixture consisting of several components (Hungarian Applications No. 2125/84 and 2869/84).

Primycin is a natural antibiotic agent originating from the fungal strain culture Thermopolyspora galeriensis, its preparation via fermentation is described in the Hungarian Patent Specification No. 153.593. Primycin is an antibiotic agent of a large spectrum, and it is firstly effective against gram positive bacteria and against polyresistent humanpathogenic strains too.

Primycin can be effectively applied for the treatment of epithelium lesions, in the urology, surgery, gynecology, dermatology, in the case of household lesions and for the treatment of burns respectively.

In the course of its application neither resistance nor, allergic reactions were experienced.

There are known further combinations of primycin with other antibiotics (Hungarian Patent Specification No. 158.241).

Although primycin is a very effective antibiotic, its therapeutical utilization was made extraordinarily difficult since it is practically insoluble in water.
Thus, several efforts had earlier been made to eliminate these difficulties and to prepare pharmaceutical compositions containing primycin in a therapeutically well utilizable form while maintaining its efficiency as a whole.

The Hungarian Patent Specification No. 173.708 publishes heterocolloidal therapeutical compositions containing primycin. Their disadvantage is the low primycin content (0.2-1.0 %). Further the ethyl alcohol used for the preparation of the heterocolloidal solution has in some cases skin irritating effect and in the case the alcohol is vaporized from the treated surface one part of the primycin is running down from the skin without exerting a biological effect. This formulation cannot be used to prepare eye-drops.

The Hungarian Patent Specification No. 194.493 describes a basic gel containing primycin and N-methyl-pyrrolidon from which therapeutical compositions and different gels were prepared. This composition having a gel consistency is useful to incorporate the active ingredient practically in all qalenic forms such as ointments, creams or foams, however it is not useful to prepare eye-drops.

The aim of our invention was to prepare a stable aqueous solution containing primycin as active ingredient, which is free from alcohol and does not contain eye-irritating substances and this way it is suitable for the preparation of eye-drops.

According to the facts set forth above the invention relates to eye-drops characterized by, that they contain
0,02 - 0,1 mass % of primycin,
15,0 - 25,0 mass % of pyrrolidon,
12,0 - 25,0 mass % of polyoxyethylene-660-hydroxystearate,
1,0 - 5,0 mass % of polyvinyl-pyrrolidon and
distilled water needed to complete to 100 mass %.

The eye-drops according to the invention are prepared in a way, that primycin is dissolved by heating in pyrrolidon-2 (Soluphor P^{R}) and to the warm solution the polyoxyethylene-660-hydroxystearate (Solutol HS 15^{R}) and polyvinyl-pyrrolidon are added. The distilled water is added to the system while stirring quickly and the solution is cooled to room-temperature while stirring.

The eye-drops according to our invention cause no eye-irritation by a 14 days treatment as it has been proved by animal experiments carried out on New Zealand rabbits.

The eye-drops according to the invention may contain beside the primycin known antimicrobic active ingredients as norfloxacin, pefloxacin, cyprofloxacin, ofloxacin too.

The compositions according to the invention are illustrated by the following Example.

### EXAMPLE

An eye-drop with the following composition is prepared
- Primycin: 0,05 mass %
- Soluphor P^{R}: 20,0 mass %
- Soluptol HS^{R}: 15,0 mass %
- Polyvinyl-pyrrolidon: 3,0 mass %
- Distilled water ad.: 100,0 mass %

## Claims

1. Eye-drop, characterized by, that it contains
0,02 - 0,1 mass % of primycin
15,0 - 25,0 mass % of pyrrolidon-2
12,0 - 25,0 mass % of polyoxyethylene-660-hydroxystearate
1,0 - 5,0 mass % of polyvinyl-pyrrolidon
and distilled water to complete to 100 mass percents.

2. Eye-drop according to claim 1, characterized by, that it contains
0,05 mass % of primycin
20,0 mass % of pyrrolidon-2
15,0 mass % of polyoxyethylene-660-hydroxystearate
3,0 mass % of polyvinyl-pyrrolidon
and distilled water to complete to 100 mass percents.

## Patentansprüche

1. Augentropfen, dadurch gekennzeichnet, daß sie enthalten:
0,02 - 0,1 Masse-% Primycin,
15,0 - 25,0 Masse-% 2-Pyrrolidon,
12,0 - 25,0 Masse-% Polyoxyethylen-660-hydroxystearat,
1,0 - 5,0 Masse-% Polyvinylpyrrolidon
und
destilliertes Wasser auf 100 Masse-%.

2. Augentropfen nach Anspruch 1, dadurch gekennzeichnet, daß sie enthalten:
0,05 Masse-% Primycin,
20,0 Masse-% 2-Pyrrolidon,
15,0 Masse-% Polyoxyethylen-660-hydroxystearat,
3,0 Masse-% Polyvinylpyrrolidon
und
destilliertes Wasser auf 100 Masse-%.

## Revendications

1. Collyre, caractérisé en ce qu'il contient
0,02 - 0,1 % en masse de primycine,
15,0 - 25,0 % en masse de pyrrolidone-2,
12,0 - 25,0 % en masse d'hydroxystéarate polyoxyéthylénique (660),
1,0 - 5,0 % en masse de polyvinylpyrrolidone
et de l'eau distillée pour compléter à 100 % en masse.

2. Collyre selon la revendication 1, caractérisé en ce qu'il contient
0,05 % en masse de primycine,
20,0 % en masse de pyrrolidone-2,
15,0 % en masse d'hydroxystéarate polyoxyéthylénique (660),
3,0 % en masse de polyvinylpyrrolidone
et de l'eau distillée pour compléter à 100 % en masse.
